(19) **Europäisches Patentamt / European Patent Office / Office européen des brevets**

(11) **EP 4 249 052 A1**

(12) 
# EUROPEAN PATENT APPLICATION
published in accordance with Art. 153(4) EPC

(43) Date of publication:
27.09.2023 Bulletin 2023/39

(21) Application number: 21885855.3

(22) Date of filing: 07.10.2021

(51) International Patent Classification (IPC):
*A61P 1/00* (2006.01)    *A61P 1/14* (2006.01)
*A61P 43/00* (2006.01)   *A61K 36/48* (2006.01)
*A23L 33/00* (2016.01)   *A23L 33/125* (2016.01)
*A23L 33/17* (2016.01)   *A61K 38/02* (2006.01)
*A61K 31/715* (2006.01)

(52) Cooperative Patent Classification (CPC):
A23L 33/00; A23L 33/125; A23L 33/17;
A61K 31/715; A61K 36/48; A61K 38/02;
A61P 1/00; A61P 1/14; A61P 43/00

(86) International application number:
PCT/JP2021/037126

(87) International publication number:
WO 2022/091736 (05.05.2022 Gazette 2022/18)

(84) Designated Contracting States:
AL AT BE BG CH CY CZ DE DK EE ES FI FR GB
GR HR HU IE IS IT LI LT LU LV MC MK MT NL NO
PL PT RO RS SE SI SK SM TR
Designated Extension States:
BA ME
Designated Validation States:
KH MA MD TN

(30) Priority: 29.10.2020 JP 2020181582

(71) Applicant: Asahimatsu Foods Co., Ltd.
Iida City, Nagano 399-2561 (JP)

(72) Inventors:
• NAKAYAMA Yasunori
Osaka-shi, Osaka 530-8605 (JP)
• TOYOHARA Kiyotsuna
Osaka-shi, Osaka 530-8605 (JP)
• ISHIGURO Takahiro
Iida City, Nagano 3992561 (JP)

(74) Representative: TBK
Bavariaring 4-6
80336 München (DE)

Remarks:
The complete document including Reference
Table(s) and the Sequence Listing(s) can be
downloaded from the EPO website

(54) **COMPOSITION FOR IMPROVING INTESTINAL BACTERIAL FLORA AND COMPOSITION FOR SUPPRESSING PRODUCTION OF SUBSTANCES BY INTESTINAL PUTREFACTION**

(57) Provided is a novel and/or improved means for improvement in intestinal bacterial flora and/or suppression of production of an intestinal putrefaction substance. A composition containing inulin and a resistant protein.

**Description**

Technical Field

The present invention relates to a composition for improvement in intestinal bacterial flora and/or suppression of production of an intestinal putrefaction substance. Specifically, the present invention relates to a composition for improvement in intestinal bacterial flora and/or suppression of production of an intestinal putrefaction substance, containing a resistant protein alone or in combination with inulin.

Background Art

Adjusting an intestinal environment is important from the viewpoint of disease prevention and health maintenance. In particular, the "back of the large intestine" (descending colon, sigmoid colon, and rectum) is an important place where many intestinal bacteria constituting intestinal bacterial flora exist. Since the "back of the large intestine" tends to be alkaline and is an environment where harmful bacteria are dominant, various intestinal putrefaction substances (ammonia, indole, and the like) produced by the harmful bacteria tend to accumulate. For this reason, the "back of the large intestine" tends to cause inflammation, which also causes various diseases. It is considered that such intestinal putrefaction substances and inflammation due to accumulation thereof are also involved in a high incidence of colorectal cancer and ulcerative colitis in the "back of the large intestine". Therefore, improvement from various aspects in the "back of the large intestine", for example, improvement in intestinal bacterial flora such as suppression of proliferation of harmful bacteria and promotion of proliferation of useful bacteria, and suppression of production of an intestinal putrefaction substance are expected to be useful for prevention of various diseases.

In recent years, various attempts have been made to suppress production of an intestinal putrefaction substance and improve intestinal bacterial flora, such as new foods and pharmaceutical products. For example, Patent Literature 1 discloses a bifidobacterium having an action of reducing an intestinal putrefaction substance. Patent Literature 2 discloses an agent for reduction of intestinal harmful bacteria, and a food and a pharmaceutical product containing the same. However, there is still room for improvement in these techniques, and in particular, expansion of variations of foods is also required.

As another means, dietary fiber has also attracted attention. Dietary fiber is one of nutritional components recommended to be taken in an amount of 18 to 20 g or more per day. In recent years, various research results have been obtained indicating that dietary fiber acts on intestinal bacteria to produce various health effects.

According to the Codex Committee on Nutrition and Foods for Special Dietary Uses (CCNFSDU) of the International Food Standards Committee (Codex Alimentarius Commission), dietary fiber is defined as follows: "Dietary fiber is regarded as polysaccharides having a degree of polymerization of 10 or more that are not digested and absorbed in the small intestine and are edible polysaccharides originally present in a food, polysaccharides obtained by physical, enzymatic, or chemical treatments, and synthesized polysaccharides, and the handling of polysaccharides having a degree of polymerization of 3 to 9 is left to the judgment of each country". However, it has been found that not only polysaccharides but also other food component residues that have not been digested in the upper gastrointestinal tract contribute deeply to the above-described action of dietary fiber on intestinal bacteria. However, the definition of "dietary fiber" by CCNFSDU described above does not include components other than these polysaccharides.

From such a viewpoint, the Japanese Association for Dietary Fiber Research proposes the term "luminacoid" as a concept including dietary fiber in a broad sense including components other than polysaccharides. According to the Japanese Association for Dietary Fiber Research, luminacoid is defined as "food components that are hard to be digested and absorbed in the small intestine of a human and exhibit physiological actions useful for maintaining health via the digestive tract". Components included in such a concept of "luminacoid" are roughly classified into non-starch-based components and starch-based components. The non-starch-based components are classified into oligosaccharides, sugar alcohols, resistant proteins, and others in addition to "dietary fiber" in a narrow sense defined by CCNFSDU. On the other hand, the starch-based components are classified into resistant starch and resistant maltodextrin.

Of particular interest among these "luminacoid" components is a "resistant protein" that refers to a protein component or a protein complex component among the non-starch-based components. Known examples of the resistant protein include soy-derived proteins such as a soy high molecular weight protein fraction (HMF) and a soy resistant protein (SRP), buckwheat-derived proteins, silk-derived proteins, and sake lees-derived proteins. As shown in Non Patent Literature 1 and 2, such a resistant protein is known to have a health-promoting effect such as cholesterol reduction.

From such findings, the resistant protein is expected to be applied to various uses as a food or beverage or a pharmaceutical product having a health-promoting action. However, there are still many unclear points regarding the detailed action and effect, and further elucidation and enhancement of the action are required for practical use.

Citation List

Patent Literature

Patent Literature 1: JP 2016-028595 A
Patent Literature 2: JP 2014-240431 A

Non Patent Literature

Non Patent Literature 1: Kato et al., Journal of Nutritional Science and Vitaminology, (2002), Vol. 48, No. 1, pp. 1-5
Non Patent Literature 2: Watanabe, Journal of the Brewing Society of Japan, 2012, Vol. 107, No. 5, pp. 282-291
Non Patent Literature 3: Nishimura et al., Soy Protein Research, Japan, 2007, Vol. 10, No. 28, pp. 48-54

Summary of Invention

Technical Problem

One of the objects of the present invention is to provide a novel and/or improved means for improvement in intestinal bacterial flora and/or suppression of production of an intestinal putrefaction substance.

Solution to Problem

As a result of intensive studies to elucidate and enhance the action of a resistant protein, the present inventors have found that when the resistant protein is used in combination with inulin, various actions of improving intestinal bacterial flora such as promotion of proliferation of Prevotella spp. and suppression of proliferation of hydrogen sulfide-producing bacteria, and an action of suppressing production of an intestinal putrefaction substance are remarkably enhanced. In addition, the present inventors have found that the resistant protein also has a heretofore unknown action of suppressing proliferation of hydrogen sulfide-producing bacteria, and have completed the present invention.

That is, the present invention provides, for example, the following embodiments.

[1] A composition containing inulin and a resistant protein.

[2] The composition according to [1], for improvement in intestinal bacterial flora.

[3] The composition according to [1] or [2], for suppression of production of an intestinal putrefaction substance.

[4] The composition according to [3], wherein the intestinal putrefaction substance is ammonia or indole.

[5] The composition according to any one of [1] to [4], for promotion of proliferation of Prevotella spp.

[6] The composition according to any one of [1] to [5], for suppression of proliferation of hydrogen sulfide-producing bacteria.

[7] A composition for suppression of proliferation of hydrogen sulfide-producing bacteria, the composition containing a resistant protein.

[8] The composition according to any one of [1] to [7], wherein the composition is a food or beverage.

[9] The composition according to any one of [1] to [7], wherein the composition is a pharmaceutical product.

[10] The composition according to [9], wherein the composition is a pharmaceutical product for oral or enteral administration.

Advantageous Effects of Invention

According to the present invention, by using a resistant protein in combination with inulin, novel and/or improved various actions of improving intestinal bacterial flora and/or actions of suppressing production of an intestinal putrefaction substance are exhibited. In addition, when the resistant protein is used alone, an action of suppressing proliferation of hydrogen sulfide-producing bacteria is also exhibited.

Brief Description of Drawings

Fig. 1 is graphs showing an ammonia concentration (μg/dL) and an indole concentration (μg/mL) in a culture supernatant obtained by an in vitro intestinal fermentation test for each sample.

Fig. 2 is graphs showing the amount of Prevotella spp. in a precipitate obtained by an in vitro intestinal fermentation test for each sample. A relative abundance (/CC) with sample CC as 1 is shown for Test 1, and a relative abundance (/IC) with sample IC as 1 is shown for Test 2.

Fig. 3 is graphs showing the amount of Desulfovibrio spp. in a precipitate obtained by an in vitro intestinal fermentation test for each sample. A relative abundance (/CC) with sample CC as 1 is shown for Test 1, and a relative abundance (/IC) with sample IC as 1 is shown for Test 2.

Description of Embodiments

Hereinafter, the present invention will be described based on specific embodiments, but the present invention is not limited to these embodiments, and can be implemented with any modifications without departing from the gist thereof.

[Summary]

As described above, a resistant protein are known to have various actions of improving an intestinal environment. On the other hand, as shown in Non Patent Literature 3, intake of the resistant protein alone promotes generation of ammonia in the intestine. In Non Patent Literature 3, it is shown that ammonia can be reduced by co-intake with pectin, but components other than pectin have not been studied much. Furthermore, components and combinations of components having an effect of reducing an intestinal putrefaction substance other than ammonia are also not well known. Here, the present inventors have conducted various studies on the resistant protein. As a result, as shown in Examples described below, it was unexpectedly found that when inulin is used in combination, an action of suppressing production of an intestinal putrefaction substance and various actions of improving intestinal bacterial flora such as promotion of proliferation of Prevotella spp. are remarkably enhanced. Furthermore, the present inventors have found that the resistant protein also has a heretofore unknown action of suppressing proliferation of hydrogen sulfide-producing bacteria. The present invention is based on such unexpected findings of the present inventors.

That is, one aspect of the present invention relates to a composition containing at least inulin and a resistant protein (hereinafter, appropriately referred to as a "first composition of the present invention" or simply as a "first composition"). The first composition of the present invention is intended to be used for improvement in intestinal bacterial flora and/or suppression of production of an intestinal putrefaction substance according to various embodiments, such as promotion of proliferation of Prevotella spp. and suppression of proliferation of hydrogen sulfide-producing bacteria.

In addition, another aspect of the present invention relates to a composition containing at least a resistant protein (hereinafter, appropriately referred to as a "second composition of the present invention" or simply as a "second composition". In addition, the first and second compositions of the present invention may be collectively referred to simply as a "composition of the present invention"). The second composition of the present invention is also used for improvement in intestinal bacterial flora and/or suppression of production of an intestinal putrefaction substance, but is intended to be used for improvement in intestinal bacterial flora and/or suppression of production of an intestinal putrefaction substance according to a specific embodiment, particularly, suppression of proliferation of hydrogen sulfide-producing bacteria.

Further, still another aspect of the present invention relates to use as a food or beverage and/or a pharmaceutical product as one embodiment of the first and/or second composition of the present invention, a method for improving intestinal bacterial flora and/or suppressing production of an intestinal putrefaction substance by administering a resistant protein alone or in combination with inulin, and the like.

[Resistant protein]

In the present invention, the "resistant protein" means, as described above, particularly a protein component or a protein complex component among non-starch-based components included in a concept of "luminacoid". In the present invention, the "luminacoid" is defined as "food components that are hard to be digested and absorbed in the small intestine of a human and exhibit physiological actions useful for maintaining health via the digestive tract" according to the definition of the Japanese Association for Dietary Fiber Research described above.

Examples of the resistant protein include proteins derived from various raw materials such as soy, buckwheat, silk, and sake lees. All of them can be used in the present invention, and among them, soy-derived proteins are preferable. Examples of the soy-derived resistant protein include a protein derived from soy itself and a protein derived from a soy processed product, and both of them can be used in the present invention. Examples of the soy processed product include Tofu, Kori-tofu, soy milk, soy flour, soy meats, soy cereals, and soy protein isolate (SPI), and any of them may be used. Specific examples of the soy-derived resistant protein include a soy high molecular weight protein fraction (HMF), a soy resistant protein (SRP), and the like, but any of them can be used in the present invention.

As the resistant protein, those isolated and purified from the above-described various raw materials may be used, but the above-described various raw materials containing a resistant protein or processed products thereof may be used as they are. In particular, when the composition of the present invention is used as a food or beverage, it is advantageous to use various raw materials containing a resistant protein or processed products thereof as most of them are in the form of a food or beverage or a food material. When the case of the soy-derived resistant protein is taken as an example, examples of the food or beverage or food material containing the soy-derived resistant protein include primary processed products such as Tofu, Kori-tofu, soy milk, soy protein isolate (SPI), soy flour, soy meats, and soy cereals, and secondary processed products such as dairy products, milk substitutes, cereals, processed meats, kneaded products, and supplements containing the primary processed products.

As a method for quantifying a resistant protein, there is no standardized quantification method like dietary fiber, but the resistant protein can be quantified by a method conforming thereto. For example, in the case where a sample to be measured is mainly composed of only lipids and proteins and hardly contains other components such as carbohydrates, the sample is subjected to a degreasing treatment, and then sequentially treated with a gastric digestive enzyme and an intestinal digestive enzyme, and the residue after the treatment is quantified as a protein component, which can be used as the amount of the resistant protein. In addition, in the case where a sample to be measured contains other non-digestible and indigestible components (e.g., "dietary fiber" in a narrow sense defined by CCNFSDU) in addition to lipids and proteins, after treatment with a gastric digestive enzyme and an intestinal digestive enzyme by the above-described method, the amount of protein components in the residue is measured by a known protein quantification method (e.g., Kjeldahl method, Dumas method, or the like), and this can be used as the amount of the resistant protein.

As an example, in the case of quantification of soy-derived resistant proteins, unique quantification methods are known for HMF and SRP, respectively. Any of these may be used, but particularly the method for quantifying SRP is more preferable as a method for quantifying a resistant protein, which is a non-digestible or indigestible protein component, since it is a method actually using a digestive enzyme. The method for quantifying SRP is a method in which a sample is subjected to a degreasing treatment, and then sequentially treated with pepsin (gastric digestive enzyme) and pancreatin (intestinal digestive enzyme) under conditions close to those of digestion of a living body, and the residue is used as the resistant protein. When a sample to be measured is Tofu, soy protein isolate, or the like, the components thereof are mainly lipids and proteins, and other components such as carbohydrates are hardly contained. Therefore, the amount of the residue obtained by the enzyme treatment described above is directly the content of the resistant protein. On the other hand, in the case of a sample containing other non-digestible and indigestible components, the amount of protein components in the residue obtained by the enzyme treatment described above may be measured by a known protein quantification method, and this may be used as the amount of the resistant protein.

Details of the method for quantifying SRP are described in Non Patent Literature 3 described above, but specifically, for example, the method can be performed by the following procedure. A sample to be measured is subjected to a degreasing treatment, then 3 g of the sample is weighed, and 30 mL of pure water is added thereto to suspend the sample. The pH of this suspension is adjusted to 1.9 with hydrochloric acid, 0.03 g of pepsin is added, and the mixture is reacted at 37°C for 5 hours. Next, the pH of this suspension is adjusted to 7.2 with sodium hydroxide, 0.09 g of pancreatin is added, and the mixture is reacted at 37°C for 14 hours. Thereafter, the enzyme is deactivated by heating at 80°C for 10 minutes, the precipitate remaining after the treatment is recovered by centrifugation, and the mass thereof is measured and used as the amount of the resistant protein. The ratio of the mass of the residue after treatment (namely, the amount of the resistant protein) to the mass of the sample before treatment is determined and used as the content of the resistant protein in the sample. If the sample also contains other non-digestible and indigestible components (such as "dietary fiber" in a narrow sense), protein components in the residue obtained by the enzyme treatment described above are quantified by a known method such as the Kjeldahl method or the Dumas method, and the content of the resistant protein in the sample is determined using the quantified protein components.

[Inulin]

In the present invention, "inulin" is a kind of "fructan" which is a polysaccharide polymer in which fructose is polymerized via a glycosidic bond and glucose is bonded to a terminal of the polymer. The fructans are classified into (1) linear fructans polymerized only with $\beta(2{\to}1)$ glycosidic bonds, (2) linear fructans polymerized only with $\beta(2{\to}6)$ glycosidic bonds, and (3) branched fructans polymerized with $\beta(2{\to}1)$ glycosidic bonds and $\beta(2{\to}6)$ glycosidic bonds according to the polymerization mode of D-fructofuranose. In the conventional nomenclature, the above (1) may be referred to as "inulin" in a narrow sense, the above (2) may be referred to as "levan", and the above (3) may be referred to as "graminan". However, in the present invention, unless otherwise specified, the branched fructans of the above (3) are also included in the definition of inulin. Furthermore, in addition to the linear fructans of the above (1) (namely, inulin in a narrow sense), among the branched fructans of the above (3), those having β(2→1) glycosidic bonds of 90% or more (namely, having β(2→6) glycosidic bonds of less than 10%) are collectively referred to as "linear inulin", and among the branched fructans of the above (3), those having β(2→1) glycosidic bonds of less than 90% (namely, having β(2→6) glycosidic bonds of 10% or more) are referred to as "branched inulin".

Examples of the linear inulin include, but are not limited to, those derived from natural materials such as Asteraceae plants such as chicory, artichoke, dandelion, and Jerusalem artichoke, and Amaryllidaceae plants such as onion, garlic, and Chinese chive. Among them, inulin derived from chicory containing high-quality inulin at a high concentration is preferable. The linear inulin can be isolated and purified from the above-described natural materials using, for example, a method for squeezing and precipitating the inulin as a crystal. In addition, commercially available linear inulin isolated and purified from such natural materials may be used. Specific examples of commercially available chicory-derived inulin include Frutafit (registered trademark) and Raftiline (registered trademark).

As the linear inulin, those synthesized by a known enzyme synthesis method or the like may be used. Specific examples of commercially available synthetic inulin include Fuji FF (registered trademark). The size of the synthetic inulin is not limited, but the chain length (degree of polymerization, which is the number of monosaccharide units to be polymerized) of each polymer molecule can be in the range of usually 3 or more, and usually 100 or less, and particularly 60 or less. The average chain length as an assembly of a large number of polymer molecules can be in the range of usually 5 or more and usually 20 or less.

Branched inulin is known to be abundantly contained in, but not limited to, for example, agave (Agavaceae), which is a succulent plant that is a raw material of tequila (e.g., J. Agric. Food Chem., (2003), 51(27): 7835-7840; J. Agric. Food Chem., (2006), 54(20): 7832-7839, and the like). The branched inulin can be isolated and purified from such a raw material as agave, for example, using a conventional method described in WO 2007/142306 A or the like. Typically, a treated product containing branched inulin (also called "agave inulin") can be obtained from piña, which is a stem part of agave, through treatments such as shredding, squeezing, filtration, purification, concentration, and powder drying.

Inulin is known to play an important role as a prebiotic. Since many animals including humans do not have an inulin hydrolase, inulin contained in an ingested food or the like reaches the large intestine without being digested and absorbed in the stomach or small intestine. Intestinal bacterial flora in the large intestine contains bacteria that secrete inulinase, which is an inulin hydrolase, and inulin that has reached the large intestine is degraded by the fermentation action of these bacteria. Since a large number of so-called good bacteria such as bifidobacteria are contained in such bacteria that secrete inulinase, the so-called good bacteria are preferentially increased by taking inulin, and propagation of so-called bad bacteria is suppressed. In addition, since an organic acid such as lactic acid is increased in the fermentation process of inulin, an intestinal regulation action also occurs.

The form of inulin is not limited, and inulin can be used in any various forms such as a powder form, a solution form, and a syrup form. When inulin derived from a natural product is used, a natural product raw material containing inulin or a processed product thereof may be used as it is. Examples of such a natural raw material include burdock, Jerusalem artichoke, and the like. On the other hand, from the viewpoint of being applied to a wide range of foods and sufficiently exhibiting a function as inulin, it is more preferable to use those extracted from a natural product. When the first or second composition of the present invention is used as a food or beverage, it may be used as a thickener or binder for a food or beverage by utilizing the thickening action of inulin.

[Composition]

The first and second compositions of the present invention contain the resistant protein described above. The content of the resistant protein is not limited, and may be appropriately adjusted according to the use and the like. According to one example, the content of the resistant protein in the first and second compositions of the present invention can be, for example, usually 0.05% by mass or more, or 0.1% by mass or more, or 0.2% by mass or more, and can be, for example, usually 25% by mass or less, or 10% by mass or less, or 5% by mass or less.

The first composition of the present invention further contains the inulin described above in addition to the resistant protein. The content of the inulin is not limited, and may be appropriately adjusted according to the use and the like. According to one example, the content of the inulin in the first composition of the present invention can be, for example, usually 0.1% by mass or more, or 0.2% by mass or more, or 0.4% by mass or more, and can be, for example, usually 50% by mass or less, or 30% by mass or less, or 10% by mass or less. Regarding the content ratio of the resistant protein to the inulin in the first composition of the present invention, the content of the inulin can be, for example, usually 0.2 parts by mass or more, or 0.5 parts by mass or more, or 1 part by mass or more, and can be, for example, usually 20 parts by mass or less, 10 parts by mass or less, or 5 parts by mass or less with respect to 1 part by mass of the resistant protein.

The form of the first and second compositions of the present invention is not particularly limited, and can be any form such as a solid, a semi-solid, a solution, a suspension, or a dispersion. Such a form of the composition can be appropriately selected according to the use of the composition and the like.

The intake amount of the first and second compositions of the present invention may be appropriately selected according to the use, type, and species, symptom, age, sex, and the like of the individual who takes the compositions. In the case of the first composition, according to one example, for a general adult, for example with a body weight of 50 kg, the weight of the resistant protein per day can be, for example, usually 0.02 g or more, or 0.05 g or more, or 0.1 g or more, and also usually 40 g or less, or 20 g or less, or 10 g or less, and the weight of the inulin can be, for example, usually 0.05 g or more, or 0.1 g or more, or 0.2 g or more, and also usually 80 g or less, or 40 g or less, or 20 g or less.

The frequency of intake of the first and second compositions of the present invention is not limited, but may be one intake, but in one embodiment, continuous intake of intestinal bacterial flora is more preferable. According to one example, the compositions can be taken, for example, usually 0.5 times a week (once every 2 weeks) or more, or 1 time or more or 2 times or more a week, and also, for example, usually 3 times a day or less, 2 times a day or less, or 1 time a day or less, and for example, the compositions can also be applied over a period of usually 1 week or more, or 2 weeks or more, or 4 weeks or more. In addition, the compositions may be taken each time, continuously taken, or intermittently taken several times a year at intervals of several months, for example.

In particular, in the case of the first composition of the present invention, the resistant protein and the inulin may of course be prepared as a single composition, but the resistant protein and the inulin may be prepared as individual independent compositions, and the individual independent compositions may be mixed immediately before intake and then taken, or the individual independent compositions may be individually taken and mixed in the body. Even in such an embodiment, it is obvious that the same action and effect as those obtained when the resistant protein and the inulin are prepared as a single composition can be obtained, and thus, such an embodiment is included in the embodiment of the first composition of the present invention.

[Use]

Both of the first and second compositions of the present invention are used for improvement in intestinal bacterial flora and/or suppression of production of an intestinal putrefaction substance. In the present invention, the "intestinal bacterial flora" means a group of bacteria that inhabits the inside of the intestine of a subject such as a human or an animal. The composition of the intestinal bacterial flora varies depending on animal species and individuals, but in a human, on average, about 30,000 kinds of bacteria and 100 trillion to 1000 trillion bacteria inhabit in the intestine to constitute the intestinal bacterial flora, and the total mass thereof reaches 1.5 to 2 kg. In the present invention, "improvement" in the intestinal bacterial flora means that by suppressing or promoting proliferation of one or two or more of bacteria contained in the intestinal bacterial flora of a subject such as a human or an animal to change the bacterial composition of the intestinal bacterial flora, or by suppressing or promoting one or two or more of actions of one or two or more of bacteria of the intestinal bacterial flora of the subject, a beneficial action for the subject is expressed, or such an action is enhanced. Examples of such improvement in the intestinal bacterial flora include, but are not limited to, promotion of proliferation of Prevotella spp. and suppression of proliferation of hydrogen sulfide-producing bacteria. In one embodiment, as a result of improvement in the intestinal bacterial flora, production of an intestinal putrefaction substance is suppressed for reasons such as a decrease in bacteria that produce an intestinal putrefaction substance such as hydrogen sulfide-producing bacteria.

In the present invention, the "intestinal putrefaction substance" means a substance harmful to a subject, which is generated as a result of metabolism by intestinal bacteria of a nutrient source such as a nitrogen source or a carbon source taken in the body in the intestine of a subject such as a human or an animal. Examples of the intestinal putrefaction substance include branched chain fatty acid (BCFA), ammonia, amine, hydrogen sulfide, phenol, indole, p-cresol, mercaptan, and skatol. It has been reported that an increase in such an intestinal putrefaction substance causes a decrease in mucus, disturbance of epithelial cells, and the like, and causes various diseases including inflammation. In one embodiment, production of an intestinal putrefaction substance is suppressed by suppressing proliferation of bacteria that produce an intestinal putrefaction substance such as hydrogen sulfide-producing bacteria.

In the present invention, "Prevotella spp." are a group of Gram-negative intestinal bacteria having high ability to degrade dietary fiber. It is known that Prevotella spp. are abundantly present in the intestine of a human who takes a long-term diet rich in indigestible starch and dietary fiber. For example, it is known that, when barley is taken, a blood glucose level is less likely to increase when the next meal is taken (this may be referred to as a "second-meal effect"), but it is clear that Prevotella spp. in the intestine is involved in such a second-meal effect.

In the present invention, "hydrogen sulfide-producing bacteria" (also referred to as "sulfate-reducing bacteria") means bacteria that reduce sulfate to sulfide ions to produce energy and generate hydrogen sulfide. Examples of the hydrogen sulfide-producing bacteria include, but are not limited to, Desulfovibrio spp., Desulfotomaculum spp., and Desulfomonas spp.

The first composition of the present invention containing a resistant protein and inulin is intended to be used for improvement in intestinal bacterial flora and/or suppression of production of an intestinal putrefaction substance according to various embodiments, such as promotion of proliferation of Prevotella spp. and suppression of proliferation of hydrogen sulfide-producing bacteria. As shown in Examples described below, when the resistant protein is used in combination with the inulin, various actions of improving intestinal bacterial flora such as promotion of proliferation of Prevotella spp. and suppression of production of an intestinal putrefaction substance are remarkably enhanced, and a synergistic effect is exhibited. Such findings are novel and unexpected findings that are neither taught nor suggested by conventional findings regarding the resistant protein and the inulin, and the first composition of the present invention is based on such novel and unexpected findings.

The second composition of the present invention containing a resistant protein is also used for improvement in intestinal bacterial flora and/or suppression of production of an intestinal putrefaction substance, but is intended to be used for improvement in intestinal bacterial flora according to a specific embodiment, particularly, suppression of proliferation of hydrogen sulfide-producing bacteria. As shown in Examples described below, the resistant protein has a heretofore unknown action of suppressing proliferation of hydrogen sulfide-producing bacteria. Such findings are novel and unexpected findings that are neither taught nor suggested by conventional findings regarding the resistant protein and the inulin, and the second composition of the present invention is based on such novel and unexpected findings.

[Food or beverage]

The composition of the present invention can also be used as a food or beverage for a human or an animal. The composition of the present invention in the form of such a food or beverage also constitutes one aspect of the present invention. The composition of the present invention in the form of a food or beverage is also referred to as a "food or beverage of the present invention". Among foods or beverages, particularly a food or beverage for an animal is also referred to as "feed".

The food or beverage of the present invention may be, but is not limited to, a food with health claims (a food for specified health uses, a food with functional claims, and the like), and in that case, it is preferable that the food or beverage of the present invention is a food with health claims for use in improvement in intestinal bacterial flora and/or suppression of production of an intestinal putrefaction substance according to various embodiments. According to one embodiment, the food or beverage of the present invention including the first composition of the present invention is preferably a food with health claims for use in promotion of proliferation of Prevotella spp. and/or suppression of proliferation of hydrogen sulfide-producing bacteria, among various embodiments of improvement in intestinal bacterial flora and/or suppression of production of an intestinal putrefaction substance. According to one embodiment, the food or beverage of the present invention including the second composition of the present invention is preferably a food with health claims for use in suppression of proliferation of hydrogen sulfide-producing bacteria, among various embodiments of improvement in intestinal bacterial flora and/or suppression of production of an intestinal putrefaction substance. When the food or beverage of the present invention is a food with health claims, the use related to improvement in intestinal bacterial flora and/or suppression of production of an intestinal putrefaction substance according to the various embodiments described above may be indicated on the product. However, the food or beverage of the present invention may of course be a food or beverage other than a food with health claims, and in this case, the food or beverage of the present invention is not disturbed to be a food or beverage taken for the purpose of improving intestinal bacterial flora and/or suppressing production of an intestinal putrefaction substance according to various embodiments, for example, promoting proliferation of Prevotella spp. and/or suppressing proliferation of hydrogen sulfide-producing bacteria. When the food or beverage of the present invention is feed intended for an animal, the use thereof is arbitrary, but it is preferable that the food or beverage is feed for use in improvement in intestinal bacterial flora and/or suppression of production of an intestinal putrefaction substance according to the various embodiments described above.

The form and type of the food or beverage of the present invention are arbitrary, and are not limited as long as a desired effect of improving intestinal bacterial flora and/or suppressing production of an intestinal putrefaction substance by an active ingredient (a resistant protein and inulin for the first composition and a resistant protein for the second composition) is exhibited. Specifically, the form of the food or beverage may be any of solid, semi-solid, and liquid (a solution, a suspension, a dispersion, a phase separation, or the like). Examples of the type of the food or beverage include tea beverages such as green tea, oolong tea, and black tea; beverages such as coffee beverages, soft beverages, jelly beverages, sports beverages, milk beverages, carbonated beverages, fruit juice beverages, lactic acid bacteria beverages, fermented milk beverages, powdered beverages, cocoa beverages, alcoholic beverages, and purified water; spreads such as butter, jam, rice seasonings, and margarine; mayonnaise, shortening, cream, dressings, bread, rice, noodles, pasta, miso soup, Tofu, milk, yogurt, soup, or sauce, bakery (bread, pies, cakes, cookies, biscuits, crackers, and the like), confectionery (biscuits, cookies, chocolate, candies, cakes, ice cream, chewing gum, tablets, and the like), and nutritional supplements (supplements having a form of pills, tablets, jellies, capsules, or the like, granola-like cereals, granola-like snake bars, cereal bars), but any form may be used. In addition to a normal food or beverage, various forms such as tablets, chewable tablets, powders, capsules, granules, drinks, gels, syrups, and liquid foods for enteral feeding can be mentioned.

The food or beverage of the present invention may further contain one or two or more of other optional components as long as a desired effect by an active ingredient (a resistant protein and inulin for the first composition and a resistant protein for the second composition) is not substantially disturbed. Examples of such other components include, but are not limited to, active ingredients such as vitamins, minerals, amino acids, and crude drugs, and various pharmaceutical additives such as various food carriers, excipients, surfactants, pH adjusting agents, stabilizers, antioxidants, dyes, flavoring agents, binders, and disintegrants. Any one of these components may be used alone, or any two or more of these components may be used in combination in any combination and ratio.

The content of each active ingredient (a resistant protein and inulin for the first composition and a resistant protein for the second composition) in the food or beverage of the present invention is not limited as long as a desired effect of improving intestinal bacterial flora and/or suppressing production of an intestinal putrefaction substance is exhibited, and can be any amount. A specific active ingredient amount may be appropriately selected according to the component composition, use, type, target for intake, and the like of the food or beverage of the present invention.

The production method of the food or beverage of the present invention is not limited, but the food or beverage of the present invention can be produced by mixing and cooking the active ingredient (a resistant protein and inulin for the first composition and a resistant protein for the second composition) together with one or two or more of other optional components according to a known food production method. A specific production method may be appropriately selected according to the component composition, use, target for intake, type, and the like of the food or beverage of the present invention. Alternatively, the food or beverage of the present invention can also be produced by blending the composition of the present invention or each active ingredient (a resistant protein and inulin for the first composition and a resistant protein for the second composition) described above with an existing food or beverage. As an example, the food or beverage of the present invention can be produced as an embodiment of the first composition containing a resistant protein and inulin by a method for impregnating, mixing, kneading, or the like a soy processed food such as Tofu or Kori-tofu containing a soy-derived resistant protein at a high concentration with natural product-derived or synthetic inulin.

In particular, in the case of the food or beverage of the present invention including the first composition of the present invention, the resistant protein and the inulin may of course be prepared as a single food or beverage, but the resistant protein and the inulin may be prepared as individual independent foods or beverages, and the individual independent foods or beverages may be mixed and cooked immediately before intake and then taken, or the individual independent foods or beverages may be individually taken and mixed in the body. Even in such an embodiment, it is obvious that the same action and effect as those obtained when the resistant protein and the inulin are prepared as a single food or beverage can be obtained, and thus, such an embodiment is included in the embodiment of the food or beverage of the present invention.

Details of the feed of the present invention are substantially the same as the details of the food or beverage of the present invention, and can be performed by appropriately applying various conditions of the food or beverage of the present invention described above.

[Pharmaceutical product]

The composition of the present invention can also be used as a pharmaceutical product for a human or an animal. The composition of the present invention in the form of such a pharmaceutical product also constitutes one aspect of the present invention. In the present description, the composition of the present invention in the form of a pharmaceutical product is also referred to as a "pharmaceutical product of the present invention".

The pharmaceutical product of the present invention is preferably, but not limited to, a pharmaceutical product for use in improvement in intestinal bacterial flora and/or suppression of production of an intestinal putrefaction substance according to various embodiments. According to one embodiment, the pharmaceutical product of the present invention including the first composition of the present invention is preferably a pharmaceutical product for use in promotion of proliferation of Prevotella spp. and/or suppression of proliferation of hydrogen sulfide-producing bacteria, among various embodiments of improvement in intestinal bacterial flora and/or suppression of production of an intestinal putrefaction substance. According to one embodiment, the pharmaceutical product of the present invention including the second composition of the present invention is preferably a pharmaceutical product for use in suppression of proliferation of hydrogen sulfide-producing bacteria, among various embodiments of improvement in intestinal bacterial flora and/or suppression of production of an intestinal putrefaction substance.

The pharmaceutical product of the present invention may further contain one or two or more of other optional components as long as a desired effect by an active ingredient (a resistant protein and inulin for the first composition and a resistant protein for the second composition) is not substantially disturbed. Examples of such other components include, but are not limited to, various pharmaceutical active ingredients, medicinal ingredients such as vitamins, minerals, amino acids, and crude drugs, and further, various pharmaceutically acceptable pharmaceutical additives such as various pharmaceutical carriers, excipients, surfactants, pH adjusting agents, stabilizers, antioxidants, dyes, flavoring agents, binders, and disintegrants. Any one of these components may be used alone, or any two or more of these components may be used in combination in any combination and ratio.

The content of each active ingredient (a resistant protein and inulin for the first composition and a resistant protein for the second composition) in the pharmaceutical product of the present invention is not limited as long as a desired effect of improving intestinal bacterial flora and/or suppressing production of an intestinal putrefaction substance is exhibited, and can be any amount. A specific active ingredient amount may be appropriately selected according to the component composition, use, administration target, administration route, dosage form, dosage regimen, and the like of the pharmaceutical product of the present invention.

The dosage form of the pharmaceutical product of the present invention is not limited, and examples thereof include oral preparations such as tablets, capsules, granules, powders, syrups, dry syrups, solutions, and suspensions; enteral preparations such as inhalants, transdermal preparations, and suppositories; and parenteral preparations such as drops and injections. In the case of a liquid preparation such as a solution or a suspension, the liquid preparation may be dissolved or suspended in water or another appropriate medium immediately before intake. In the case of a tablet, a granule, or the like, the surface thereof may be coated by a well-known method. In the case of an injection, it may be in the form of a solution of sterile distilled water or sterile physiological saline containing a solubilizing agent as necessary. A specific dosage form may be appropriately selected according to the component composition, use, administration target, administration route, dosage regimen, and the like of the pharmaceutical product of the present invention.

In particular, in the case of the pharmaceutical product of the present invention including the first composition of the present invention, the resistant protein and the inulin may of course be prepared as a single pharmaceutical product, but the resistant protein and the inulin may be prepared as individual independent pharmaceutical products, and the individual independent pharmaceutical products may be mixed immediately before administration and then administered, or the individual independent pharmaceutical products may be individually administered and mixed in the body. Even in such an embodiment, it is obvious that the same action and effect as those obtained when the resistant protein and the inulin are prepared as a single pharmaceutical product can be obtained, and thus, such an embodiment is included in the embodiment of the pharmaceutical product of the present invention.

The production method of the pharmaceutical product of the present invention is not limited, but the pharmaceutical product of the present invention can be produced by mixing and formulating the active ingredient (a resistant protein and inulin for the first composition and a resistant protein for the second composition) together with one or two or more of other optional components according to a known formulation method. A specific production method of the pharmaceutical product of the present invention may be appropriately selected according to the component composition, use, administration target, administration route, dosage form, dosage regimen, and the like of the pharmaceutical product of the present invention.

The administration route of the pharmaceutical product of the present invention is not limited, but is preferably an administration route in which a desired effect of improving intestinal bacterial flora and/or suppressing production of an intestinal putrefaction substance is sufficiently exhibited. Examples thereof include oral as well as enteral administration (transluminal administration, enteral injection administration, and the like). A specific administration route may be appropriately selected according to the component composition, use, administration target, dosage form, dosage regimen, and the like of the pharmaceutical product of the present invention. In the pharmaceutical product of the present invention including the first composition of the present invention described above, when the resistant protein and the inulin are administered to a subject as individual independent pharmaceutical products, they may be administered to the subject by the same route, or may be administered to the subject by different routes.

The dosage regimen of the pharmaceutical product of the present invention is also not limited, and an appropriate dosage regimen may be appropriately selected according to the component composition, use, administration target, dosage form, administration route, and the like of the pharmaceutical product of the present invention. In the pharmaceutical product of the present invention including the first composition of the present invention described above, when the resistant protein and the inulin are administered to a subject as individual independent pharmaceutical products, they may be administered to the subject in the same dosage regimen, or may be administered to the subject in different dosage regimens at different timings.

[Others]

Although various aspects of the present invention have been described above, the present invention is not limited to these aspects. As will be apparent to those skilled in the art, it is also possible to extract another arbitrary aspect of the present invention from the above detailed description and the description of Examples and the like described below.

For example, by administering an active ingredient (a resistant protein and inulin in the case of an embodiment corresponding to the first composition, and a resistant protein in the case of an embodiment corresponding to the second composition) to a subject such as a human or an animal in need thereof, it is also possible to cause action and effect such as improvement in intestinal bacterial flora and/or suppression of production of an intestinal putrefaction substance according to various embodiments, for example, promotion of proliferation of Prevotella spp. and/or suppression of proliferation of hydrogen sulfide-producing bacteria in the subject. Such a method for improving intestinal bacterial flora and/or a method for suppressing production of an intestinal putrefaction substance (e.g., a method for promoting proliferation of Prevotella spp. and/or a method for suppressing proliferation of hydrogen sulfide-producing bacteria) also constitute one aspect of the present invention. As is apparent from the above description, particularly as an embodiment corresponding to the first composition of the present invention, when the resistant protein and the inulin are administered to a subject, the resistant protein and the inulin may be administered to the subject together, or the resistant protein and the inulin may be administered to the subject individually. Details thereof are also as described above.

Use of an active ingredient (a resistant protein and inulin for the first composition and a resistant protein for the second composition) in the production of the composition of the present invention, particularly the pharmaceutical product or the food or beverage of the present invention also constitutes one aspect of the present invention. Details thereof are also as described above.

In addition, particularly as an embodiment corresponding to the first composition of the present invention, a method for enhancing the action of improving intestinal bacterial flora of an existing pharmaceutical product or food or beverage and/or a method for suppressing production of an intestinal putrefaction substance by combining the existing pharmaceutical product or food or beverage containing one of the resistant protein and the inulin with the other of the resistant protein and the inulin also constitute one aspect of the present invention. Details thereof are also as described above. Examples

Hereinafter, the present invention will be described in more detail with reference to Examples, but these Examples are merely examples shown for convenience of description, and the present invention is not limited to these Examples in any sense.

[Materials and methods]

• Preparation of resistant protein:

Soy (name of variety: BS1512, produced in America), Tofu (obtained by adding calcium chloride to soy milk obtained by immersing, grinding, heating, and filtering soy and compression-molding the mixture), and Kori-tofu ("Shin Asahi Tofu" manufactured by Asahimatsu Foods Co., Ltd.) were used as protein sources. The protein sources were degreased and then suspended in water, and the pH of the suspension was adjusted to 1.8 to 2.0 with hydrochloric acid. Then, 1% by mass of pepsin was added to the protein sources, and the mixture was treated at 37°C for 5 hours. After the reaction, the pH of the reaction solution was adjusted to 7.2 with sodium hydrogen carbonate, 3% by mass of pancreatin of the protein sources was added, and the reaction was carried out at 37°C for 14 hours. After the reaction, the pH of the reaction solution was adjusted to acidity with hydrochloric acid, and after centrifugation, the obtained precipitated fraction was suspended in water, and the pH of the suspension was adjusted to 7.0 with sodium hydrogen carbonate. This suspension was desalted, and the resulting powder after lyophilization was used as resistant proteins (soy RP, Tofu RP, and Kori-tofu RP, respectively).

• Preparation of sample:

Using each resistant protein (soy RP, Tofu RP, and Kori-tofu RP) obtained in the above procedure, each sample having the composition described in Table 1 below was prepared. % shown in Table 1 means "% by mass". As the cellulose shown in Table 1, one purchased from FUJIFILM Wako Pure Chemical Corporation was used, Frutafit IQ (registered trademark) (manufactured by Sensus) was used as the inulin, and Nutrient Broth (manufactured by Difco) was used as the control protein.

[Table 1]

| Test | Sample | Carbon source (1.5%) | Nitrogen source (0.8%) |
|---|---|---|---|
| Test 1 | CC | Cellulose | Control protein |
| | IC | Inulin | Control protein |
| | CK | Cellulose | Kori-tofu RP |
| | IK | Inulin | Kori-tofu RP |
| Test 2 | IC | Inulin | Control protein |
| | ID | Inulin | Soy RP |

(continued)

| Test | Sample | Carbon source (1.5%) | Nitrogen source (0.8%) |
|------|--------|----------------------|------------------------|
|      | IT     | Inulin               | Tofu RP                |
|      | IK     | Inulin               | Kori-tofu RP           |

• Preparation of fecal suspension:

An in vitro intestinal fermentation test using feces of pigs (4 to 5 months old) was employed to reproduce intestinal bacterial flora imitating the human large intestine. Four-fold physiological saline was added to and mixed with pig feces, and the mixture was filtered using a mesh, and the mixture was frozen and stored as a fecal suspension until use.

• In vitro intestinal fermentation test:

The in vitro intestinal fermentation test was performed using a Bio Jr. 8 culture apparatus (ABLE Corporation) according to the following procedure. A culture tank to which 90 mL of distilled water was added was heat-sterilized at 121°C for 20 minutes. After cooling, 10 mL of the fecal suspension was put in the culture tank and mixed, and the mixture was cultured overnight. Setting conditions of the culture apparatus were a temperature of 37°C and a stirring bar rotation speed of 400 rpm. The culture apparatus was set such that when the pH in the culture tank fell below 5.5, an alkali solution (2 M sodium hydroxide) was automatically added dropwise. In addition, in order to keep the inside of the culture tank in an anaerobic state, the culture tank was filled with carbon dioxide gas. After overnight culture, each sample shown in Table 1 described above was added to the culture tank together with 20 mL of distilled water (Otsuka distilled water, Otsuka Pharmaceutical Co., Ltd.). After 48 hours of culture, 1 mL of the collected culture supernatant was centrifuged (10,000 rpm, 4°C, 5 minutes) to obtain a culture supernatant and a precipitate. This in vitro intestinal fermentation test was performed in two separate tests of Test 1 and Test 2 shown in Table 1 described above. For each sample, the ammonia concentration, the indole concentration, the amount of specific bacteria, and the amount of short-chain fatty acid were measured by the following procedure using the culture supernatant and the precipitate obtained after the test.

• Measurement of ammonia concentration:

For each sample, the ammonia concentration was measured according to the following procedure using the culture supernatant obtained after the test. The culture supernatant was diluted with distilled water, and the ammonia concentration was measured by a colorimetric method based on absorbance at 630 nm according to the instruction included in the kit using an ammonia kit for blood test "Ammonia Test Wako" (FUJIFILM Wako Pure Chemical Corporation). The colorimetric calibration curve was prepared using standard solutions for calibration curve obtained by serially diluting the ammonia standard solution contained in the kit so as to have concentrations of 0, 100, 200, 300, and 400 $\mu$g/dL.

• Measurement of indole concentration:

For each sample, the indole concentration was measured according to the following procedure using the culture supernatant obtained after the test. Indole (FUJIFILM Wako Pure Chemical Corporation) was dissolved in a 0.1 M phosphate buffer (pH 5.5) to prepare a 100 $\mu$g/mL indole standard solution. This was serially diluted with a 0.1 M phosphate buffer (pH 5.5) so as to have concentrations of 0, 0.195, 0.781, 3.125, 12.5, and 50 $\mu$g/mL to prepare standard solutions for calibration curve. A coloring solution was prepared by dissolving 1.47 g of p-dimethylaminobenzaldehyde (FUJIFILM Wako Pure Chemical Corporation) in 100 mL of sulfuric acid alcohol (5.2 mL of 95% sulfuric acid + 94.8 mL of 95% ethanol). 1 mL of the coloring solution was added to 200 $\mu$L of each culture supernatant and each standard solution for calibration curve, the mixture was allowed to stand for 20 minutes, then centrifuged (3,000 rpm, 4°C, 10 minutes), the absorbance of each culture supernatant at 568 nm was measured, and the color was compared with that of the standard solution for calibration curve to determine the indole concentration of each culture supernatant.

• Measurement of amount of bacteria:

For each sample, using the precipitate obtained after the test, the amount of each of Prevotella spp. and Desulfovibrio spp. (hydrogen sulfide-producing bacteria) were measured by the following procedure using a real-time PCR method. DNA was extracted from each precipitate using NucleoSpin DNA Stool (MACHEREY-NAGEL GmbH & Co. KG). For the extracted DNA solution, the base sequence for detection in each bacterial DNA was amplified by Fast SYBR Green Master Mix (Thermo Fisher Scientific, Inc.) and CFX384 Touch Real-Time Detection System (Bio-Rad Laboratories, Inc.) using each bacterial detection primer having the base sequence shown in Table 2 below, and the amount of each bacterium was determined.

[Table 2]

| | Forward primer | Reverse primer |
|---|---|---|
| Prevotella | CACRGTAAACGATGGATGCC (SEQ ID NO: 1) | CGTCGGGTTGCAGACC (SEQ ID NO: 2) |
| Desulfovibrio | CCGTAGATATCTGGAGGAACATCAGA (SEQ ID NO: 3) | ACATCTAGCATCCATCGTTTACAGC (SEQ ID NO: 4) |

[Results and discussion]

• Results:

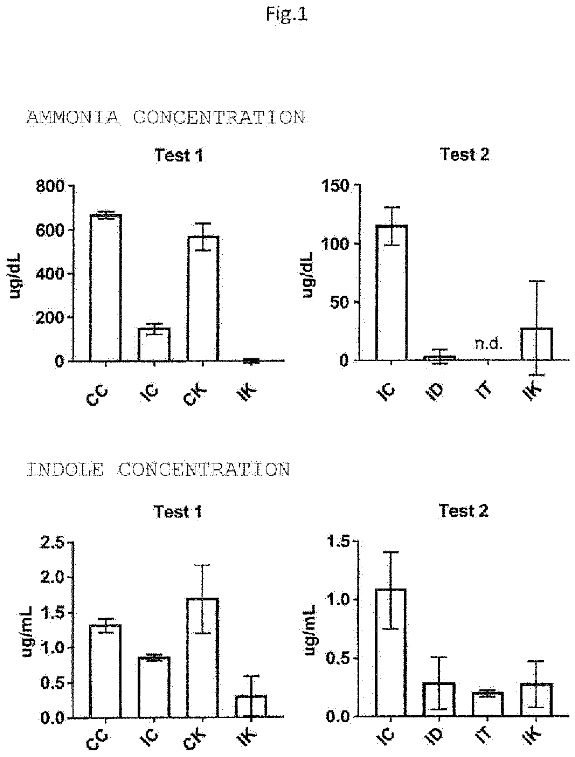

The ammonia concentration, the indole concentration, and the amount of specific bacteria obtained for each sample by the above tests and measurements are shown in Table 3 below and attached Figs. 1 to 3.

[Table 3]

| | Test 1 | | | | Test 2 | | | |
|---|---|---|---|---|---|---|---|---|
| | CG | IC | CK | IK | IC | ID | IT | IK |
| Ammonia ($\mu$g/dL) | 671.2 | 151.8 | 572.6 | 4.4 | 115.9 | 3.9 | 0.0 | 27.9 |
| Indole ($\mu$g/mL) | 1.34 | 0.87 | 1.70 | 0.31 | 1.09 | 0.29 | 0.21 | 0.28 |
| Prevotella (relative abundance) | 1.00 | 0.70 | 0.78 | 4.57 | 1.00 | 3.33 | 2.01 | 2.38 |
| Desulfovibrio (relative abundance) | 1.000 | 0.008 | 0.132 | 0.006 | 1.000 | 0.513 | 0.246 | 0.101 |

• Ammonia concentration and indole concentration:

When the results of the ammonia concentration and the indole concentration shown in Table 3 above and the graphs of attached Fig. 1 are examined, first, according to Test 1, in the IK group containing Kori-tofu RP together with inulin, decreases in the ammonia concentration and the indole concentration were observed as compared with the IC group containing inulin but not containing Kori-tofu RP or the CK group containing Kori-tofu RP but not containing inulin. Here, a negative synergistic effect of the combination of inulin and Kori-tofu RP was calculated by the following formula (1).
[Mathematical Formula 1]

$$[\text{Negative synergistic effect}] = (CC - IK)/\{(CC - IC)/2 + (CC - CK)/2\} \quad (1)$$

When calculated according to the above formula (1), the negative synergistic effect of the IK group for ammonia concentration was (671.2 - 4.4)/ f (671.2 - 151.8)/2 + (671.2 - 572.6)/2} = 2.16 times. The negative synergistic effect of the IK group for indole concentration was (1.34 - 0.31)/{(1.34 - 0.87)/2 + (1.34 - 1.70)/2} = 18.72 times. That is, the negative synergistic effect in the IK group was confirmed for both the ammonia concentration and the indole concentration. Therefore, it is found that production of intestinal putrefaction substances (ammonia and indole) was suppressed by the combination of inulin and Kori-tofu RP. In particular, from the comparison between the IK group and the CK group, it is found that the action of suppressing production of intestinal putrefaction substances (ammonia and indole) by Kori-tofu RP is greatly enhanced by combining inulin, and the synergistic effect is obtained.
Next, according to Test 2, in the ID group, the IT group, and the IK group containing soy RP, Tofu RP, and Kori-tofu RP together with inulin, respectively, decreases in ammonia concentration and indole concentration were observed as compared with the IC group containing inulin but not containing a resistant protein. Therefore, it was shown that when not only Kori-tofu RP but also soy RP and Tofu RP were used as resistant proteins, production of ammonia and indole was suppressed by the combination with inulin.

• Amount of Prevotella spp.:

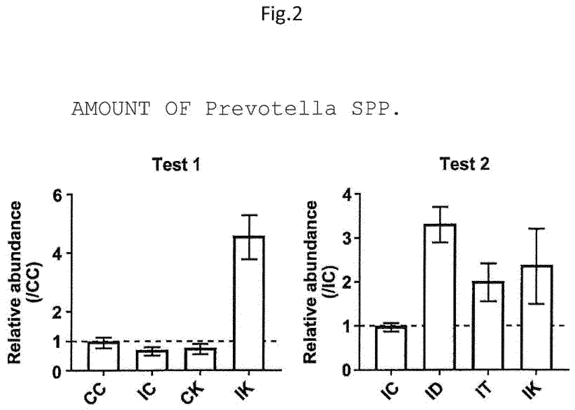

When the results of the amount of Prevotella spp. shown in Table 3 above and the graphs of attached Fig. 2 are examined, first, according to Test 1, in the IK group containing Kori-tofu RP together with inulin, an increase in the amount of Prevotella spp. was observed as compared with the IC group containing inulin but not containing Kori-tofu RP or the CK group containing Kori-tofu RP but not containing inulin. Here, a positive synergistic effect of the combination of inulin and Kori-tofu RP was calculated by the following formula (2).
[Mathematical Formula 2]

$$[\text{Positive synergistic effect}] = \text{IK}/(\text{IC}/2 + \text{CK}/2) \qquad (2)$$

When calculated according to the above formula (2), the positive synergistic effect of the IK group was 4.57/(0.70/2 + 0.78/2) = 6.18 times from each value shown in Table 3. Therefore, a positive synergistic effect in the IK group was confirmed, and it was shown that proliferation of Prevotella spp. was promoted by the combination of inulin and Kori-tofu RP. In particular, from the comparison between the IK group and the CK group, it is found that the action of promoting proliferation of Prevotella spp. by Kori-tofu RP is greatly enhanced by combining inulin, and the synergistic effect is obtained.

Next, according to Test 2, in the ID group, the IT group, and the IK group containing soy RP, Tofu RP, and Kori-tofu RP together with inulin, respectively, an increase in Prevotella spp. was observed as compared with the IC group containing inulin but not containing a resistant protein. Therefore, it was shown that when not only Kori-tofu RP but also soy RP and Tofu RP were used as resistant proteins, proliferation of Prevotella spp. was promoted by the combination with inulin.

• Amount of Desulfovibrio spp.:

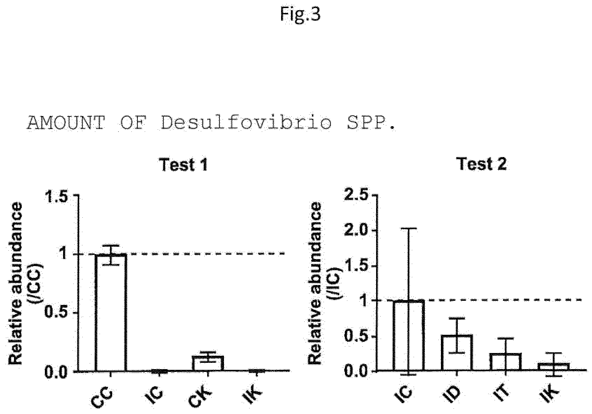

According to the results of the amount of Desulfovibrio spp. shown in Table 3 above and the graphs of attached Fig. 3, in Test 1, a decrease in the amount of Desulfovibrio spp. was observed in all of the IC group containing inulin but not containing a resistant protein, the CK group containing Kori-tofu RP but not containing inulin, and the IK group containing both Kori-tofu RP and inulin, as compared with the CC group containing neither inulin nor a resistant protein. In addition, in Test 2, as compared with the IC group containing inulin but not containing a resistant protein, in the ID group, the IT group, and the IK group containing soy RP, Tofu RP, and Kori-tofu RP together with inulin, respectively, a decrease in Desulfovibrio spp. in the culture supernatant was observed. Therefore, it was shown that proliferation of hydrogen sulfide-producing bacteria (Desulfovibrio spp.) was suppressed by resistant proteins (Kori-tofu RP, soy RP, and Tofu RP).

Industrial Applicability

The present invention has extremely high applicability in an industrial field in which an action of improving intestinal bacterial flora and/or an action of suppressing production of an intestinal putrefaction substance is required, particularly in the fields of pharmaceutical products and foods or beverages.

**Claims**

1. A composition comprising inulin and a resistant protein.

2. The composition according to claim 1, for improvement in intestinal bacterial flora.

3. The composition according to claim 1 or 2, for suppression of production of an intestinal putrefaction substance.

4. The composition according to claim 3, wherein the intestinal putrefaction substance is ammonia or indole.

5. The composition according to any one of claims 1 to 4, for promotion of proliferation of Prevotella spp.

6. The composition according to any one of claims 1 to 5, for suppression of proliferation of hydrogen sulfide-producing bacteria.

7. A composition for suppression of proliferation of hydrogen sulfide-producing bacteria, the composition comprising a resistant protein.

8. The composition according to any one of claims 1 to 7, wherein the composition is a food or beverage.

9. The composition according to any one of claims 1 to 7, wherein the composition is a pharmaceutical product.

10. The composition according to claim 9, wherein the composition is a pharmaceutical product for oral or enteral administration.

Fig.1

AMMONIA CONCENTRATION

INDOLE CONCENTRATION

Fig.2

AMOUNT OF Prevotella SPP.

Fig.3

AMOUNT OF Desulfovibrio SPP.

**Test 1**

**Test 2**

## INTERNATIONAL SEARCH REPORT

| International application No. |
|---|
| **PCT/JP2021/037126** |

**A. CLASSIFICATION OF SUBJECT MATTER**

*A61P 1/00*(2006.01)i; *A61P 1/14*(2006.01)i; *A61P 43/00*(2006.01)i; *A61K 36/48*(2006.01)i; *A23L 33/00*(2016.01)i; *A23L 33/125*(2016.01)i; *A23L 33/17*(2016.01)i; *A61K 38/02*(2006.01)i; *A61K 31/715*(2006.01)i
FI: A23L33/00; A23L33/125; A23L33/17; A61K38/02; A61K36/48; A61K31/715; A61P1/00; A61P1/14; A61P43/00 121

According to International Patent Classification (IPC) or to both national classification and IPC

**B. FIELDS SEARCHED**

Minimum documentation searched (classification system followed by classification symbols)

A61P1/00; A61P1/14; A61P43/00; A61K36/48; A23L33/00; A23L33/125; A23L33/17; A61K38/02; A61K31/715

Documentation searched other than minimum documentation to the extent that such documents are included in the fields searched

Published examined utility model applications of Japan 1922-1996
Published unexamined utility model applications of Japan 1971-2021
Registered utility model specifications of Japan 1996-2021
Published registered utility model applications of Japan 1994-2021

Electronic data base consulted during the international search (name of data base and, where practicable, search terms used)

JSTPlus/JMEDPlus/JST7580 (JDreamIII); CAplus/REGISTRY/FSTA/CABA/AGRICOLA/MEDLINE/EMBASE/BIOSIS (STN); Scopus

**C. DOCUMENTS CONSIDERED TO BE RELEVANT**

| Category* | Citation of document, with indication, where appropriate, of the relevant passages | Relevant to claim No. |
|---|---|---|
| X | CN 110179108 A (SHANGHAI POLARIS HEALTH CO., LTD.) 30 August 2019 (2019-08-30)<br>claims, paragraphs [0023], [0030], [0088], examples, fig. 1-23 | 1-10 |
| A | JP 2013-510865 A (NESTEC S.A) 28 March 2013 (2013-03-28)<br>claims, paragraph [0171] | 5 |
| A | JP 2018-50526 A (TEIJIN LTD) 05 April 2018 (2018-04-05)<br>claims, paragraph [0009] | 5 |
| X | 荻田　佑ほか, ダイズ難消化性タンパク質の腸管バリア増強効果, 日本農芸化学会２０１８年度大会講演要旨集, 05 March 2018, Lecture no. 2A13p13<br>entire text, (OGITA, Tasuku et al.), non-official translation (Intestinal barrier enhancement effect of soybean resistant protein. Lecture abstracts of the 2018 Annual Meeting of Japan Society for Bioscience, Biotechnology, and Agrochemistry.) | 7-10 |

☑ Further documents are listed in the continuation of Box C.    ☑ See patent family annex.

| * | Special categories of cited documents: | "T" | later document published after the international filing date or priority date and not in conflict with the application but cited to understand the principle or theory underlying the invention |
|---|---|---|---|
| "A" | document defining the general state of the art which is not considered to be of particular relevance | "X" | document of particular relevance; the claimed invention cannot be considered novel or cannot be considered to involve an inventive step when the document is taken alone |
| "E" | earlier application or patent but published on or after the international filing date | | |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | "&" | document member of the same patent family |
| "P" | document published prior to the international filing date but later than the priority date claimed | | |

| Date of the actual completion of the international search | Date of mailing of the international search report |
|---|---|
| **26 November 2021** | **07 December 2021** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | |
| | Telephone No. |

Form PCT/ISA/210 (second sheet) (January 2015)

## INTERNATIONAL SEARCH REPORT

| International application No. |
| --- |
| **PCT/JP2021/037126** |

### C. DOCUMENTS CONSIDERED TO BE RELEVANT

| Category* | Citation of document, with indication, where appropriate, of the relevant passages | Relevant to claim No. |
| --- | --- | --- |
| X | WANG, X. et al. Prebiotic supplementation of In Vitro fecal fermentations inhibits proteolysis by gut bacteria, and host diet shapes gut bacterial metabolism and response to intervention. Applied and Environmental Microbiology. 18 April 2019, vol. 85, no. 9, pp. 1-14, DOI: 10.1128/aem.02749-18<br>abstract, materials and methods, fig. 2-6 | 1-10 |
| X | JP 2017-508450 A (LU, Mingfu) 30 March 2017 (2017-03-30)<br>claims, paragraph [0001], example 1 | 1, 8-10 |
| A | JP 2020-130093 A (TEIJIN LTD) 31 August 2020 (2020-08-31)<br>entire text, all drawings | - |
| A | JP 2006-169204 A (MARUTOMO CO LTD) 29 June 2006 (2006-06-29)<br>entire text, all drawings | - |
| A | WO 2018/056284 A1 (MEIJI CO LTD) 29 March 2018 (2018-03-29)<br>entire text, all drawings | - |
| A | 上野 豊・石黒貴寛, 大豆の凍結変性による難消化性たん白質のプレバイオティック機能性強化, 大豆たん白質研究, 30 May 2016, vol. 19, pp. 50-54<br>entire text, (UENO, Yutaka. ISHIGURO, Takahiro. Availability of Soybean Resistant Protein Derived from Frozen Soybean Curd for Manifesting Specific Groups of Animal Intestinal Microorganisms. Soy protein research, Japan.) | - |

Form PCT/ISA/210 (second sheet) (January 2015)

**INTERNATIONAL SEARCH REPORT**

International application No.

**PCT/JP2021/037126**

| Box No. I | Nucleotide and/or amino acid sequence(s) (Continuation of item 1.c of the first sheet) |
|---|---|

1. With regard to any nucleotide and/or amino acid sequence disclosed in the international application, the international search was carried out on the basis of a sequence listing:

    a. ☑ forming part of the international application as filed:

        ☑ in the form of an Annex C/ST.25 text file.

        ☐ on paper or in the form of an image file.

    b. ☐ furnished together with the international application under PCT Rule 13*ter*.1(a) for the purposes of international search only in the form of an Annex C/ST.25 text file.

    c. ☐ furnished subsequent to the international filing date for the purposes of international search only:

        ☐ in the form of an Annex C/ST.25 text file (Rule 13*ter*.1(a)).

        ☐ on paper or in the form of an image file (Rule 13*ter*.1(b) and Administrative Instructions, Section 713).

2. ☐ In addition, in the case that more than one version or copy of a sequence listing has been filed or furnished, the required statements that the information in the subsequent or additional copies is identical to that forming part of the application as filed or does not go beyond the application as filed, as appropriate, were furnished.

3. Additional comments:

Form PCT/ISA/210 (continuation of first sheet) (January 2015)

## INTERNATIONAL SEARCH REPORT
### Information on patent family members

International application No.

**PCT/JP2021/037126**

| Patent document cited in search report | | | Publication date (day/month/year) | Patent family member(s) | | | Publication date (day/month/year) |
|---|---|---|---|---|---|---|---|
| CN | 110179108 | A | 30 August 2019 | (Family: none) | | | |
| JP | 2013-510865 | A | 28 March 2013 | US | 2012/0269865 | A1 | |
| | | | | claims, paragraph [0200] | | | |
| | | | | US | 2016/0100617 | A1 | |
| | | | | WO | 2011/060123 | A1 | |
| | | | | EP | 2498626 | A1 | |
| | | | | CA | 2777941 | A1 | |
| | | | | CN | 102595934 | A | |
| | | | | AU | 2010319490 | A | |
| | | | | MX | 2012004778 | A | |
| | | | | RU | 2012123959 | A | |
| | | | | CN | 104206946 | A | |
| | | | | DK | 2498626 | T | |
| | | | | ES | 2545597 | T | |
| | | | | HK | 1172516 | A | |
| | | | | BR | 112012011294 | A | |
| | | | | IN | 3632DEN2012 | A | |
| | | | | JP | 2016-166176 | A | |
| JP | 2018-50526 | A | 05 April 2018 | (Family: none) | | | |
| JP | 2017-508450 | A | 30 March 2017 | US | 2015/0140178 | A1 | |
| | | | | claims, paragraph [0041] | | | |
| | | | | US | 2016/0324204 | A1 | |
| | | | | WO | 2016/019597 | A1 | |
| | | | | EP | 3138414 | A1 | |
| | | | | DE | 102015100923 | A1 | |
| | | | | CN | 104187624 | A | |
| | | | | AU | 2014403009 | A | |
| | | | | CA | 2944529 | A1 | |
| | | | | MX | 2016008835 | A | |
| | | | | RU | 2016132172 | A | |
| | | | | BR | 112016016588 | A | |
| JP | 2020-130093 | A | 31 August 2020 | (Family: none) | | | |
| JP | 2006-169204 | A | 29 June 2006 | (Family: none) | | | |
| WO | 2018/056284 | A1 | 29 March 2018 | TW | 201822649 | A | |

Form PCT/ISA/210 (patent family annex) (January 2015)

22

**EP 4 249 052 A1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- JP 2016028595 A
- JP 2014240431 A

- WO 2007142306 A

### Non-patent literature cited in the description

- **KATO et al.** *Journal of Nutritional Science and Vitaminology,* 2002, vol. 48 (1), 1-5
- **WATANABE.** *Journal of the Brewing Society of Japan,* 2012, vol. 107 (5), 282-291
- **NISHIMURA et al.** *Soy Protein Research, Japan,* 2007, vol. 10 (28), 48-54

- *J. Agric. Food Chem.,* 2003, vol. 51 (27), 7835-7840
- *J. Agric. Food Chem.,* 2006, vol. 54 (20), 7832-7839